# EUROPEAN PATENT SPECIFICATION

(11) **EP 1 501 599 B1**
(45) Date of publication and mention of the grant of the patent: **27.05.2009**
(21) Application number: 03747549.8
(22) Date of filing: 28.03.2003
(51) Int. Cl.: A61N 1/375

(54) **DEVICE IN CONNECTION WITH PACERS**
VORRICHTUNG IN VERBINDUNG MIT SCHRITTMACHERN
DISPOSITIF CONNECTE A DES STIMULATEURS CARDIAQUES

(30) Priority: 30.04.2002 SE 0201322
(43) Date of publication of application: 02.02.2005
(73) Proprietor: St Jude Medical AB, 175 84 Järfälla (SE)
(72) Inventor: HÖRNFELDT, Martin, S-113 38 STOCKHOLM (SE); SKOOG ANDERSSON, Eva, S-135 69 Tyresö (SE)
(74) Representative: Kalling, Sven Owe
(86) International application number: PCT/SE2003/000513
(87) International publication number: WO 2003/092808

(56) References cited:
- US-A- 3 908 668
- US-A- 4 072 154
- US-A- 4 154 248
- US-A- 5 507 662
- US-A- 5 679 026
- US-A- 5 683 433

## Description

### Technical field of the invention.

The present invention relates to pacer housings and more particularly to those parts of the housing intended for connection to the electrode leads.

### Background of the invention.

Implantable pacers normally comprise a pacer housing (also called can) containing electronic circuitry and a unit for electric power as well as different electrodes which are connected to the interior parts in the pacer housing and which are to be implanted in or in the vicinity of the heart. The electrodes are connected to the pacer by means of leads. The internal parts of the pacers have to be well protected against the internal environment, especially the body fluids in the body for a long period of time, which places strict requirements on all entries into the interior of the can and especially on the connections of the leads to the housing. At the same time it should be possible to disconnect the pacer from the implanted leads for replacement or servicing of the pacer. The connective parts of the pacer and the leads have largely been standardized so as to encompass a relatively deep female socket comprising a number of contact surfaces whereas the leads are provided with a male part comprising one or several corresponding peripheral, generally circular contact surfaces.

At present the connective part of the pacer housing containing the female socket is made of a transparent material, normally of epoxy resin, which is molded onto the housing and onto contacts extending outwardly from the housing. The male part of the leads is normally locked by means of set screws, although other fastening means have been envisaged. The positioning and alignment of the different contact surfaces and of the fastening means or metallic threads for the set screws prior to the molding of the connective part is however complicated and the delay in the manufacturing process incurred by the curing of the epoxy resin is considerable. It would thus be desirable if the molding procedure could be dispensed with. It should however be noted that the molded connective part allows visible confirmation that the lead connector is adequately inserted.

It has been discussed that these complexities could be avoided by designing the pacer with a socket located inside the metal housing. To our knowledge this kind of sockets, sometimes termed "black holes", are not used at present.

US-A-4,934,366 and US-A-5,324,311 describe two interior sockets or black holes for pacers. Both designs comprise a tubular member consisting of a number of longitudinally alternating sections made of metal respectively of insulating ceramics. An end section of metal can be welded or bonded to an opening in the pacer housing by means of a flange. The use of different materials however set high standards in regard of precision and durability of the component parts as well as on the assembly procedure thereof. This is especially important since the interior sockets must meet very high standards regarding the integrity of the interior of the pacer housing during long times of implantation in a demanding environment.
WO 00/12174, on which the preamble of claim 1 is based, describes a tubular connector which is welded in both ends to the pacer encapsulation.

### Short description of the inventive concept

According to the invention a tubular connector as disclosed in WO 00/12174 is used in an implantable pacemaker. The connector is designed such that a part of the most proximal lead contact pin extends beyond the second opening of said tubular connector when the lead is adequately inserted in the tubular connector. A transparent device is glued on the pacemaker so that the portion of the lead contact pin that extends beyond the second opening of the tubular connector is at least partly surrounded by the transparent device. This allows for a visual confirmation that the lead is adequately inserted into the connector. A molding process on the pacemaker housing will however not be necessary. According to the invention a prefabricated transparent device is e.g. glued to a pacer housing comprising a tubular connector which is open in both ends in accordance with the appended main claim. Preferred embodiments are set forth in the dependent claims.

### Short description of the appended drawings

Fig 1 shows a conventional pacer housing with a transparent, molded connective part;
Fig 2 shows a lead with a male connective part;
Fig 3 shows a preferred embodiment in in accordance with the present invention which provides visual confirmation that the electrode lead is adequately inserted into the pulse generator.
Fig 4 shows a preferred embodiment in which a different design of the tubular connector is showed.
Fig 5 shows an embodiment in which the pacemaker is built using functional modules that also contribute to the outer enclosure of the pacemaker.
Fig 6 shows an embodiment which provides visual confirmation that the electrode lead is adequately inserted into the pulse generator combined with a locking mechanism.

### Detailed description of preferred embodiments of the invention.

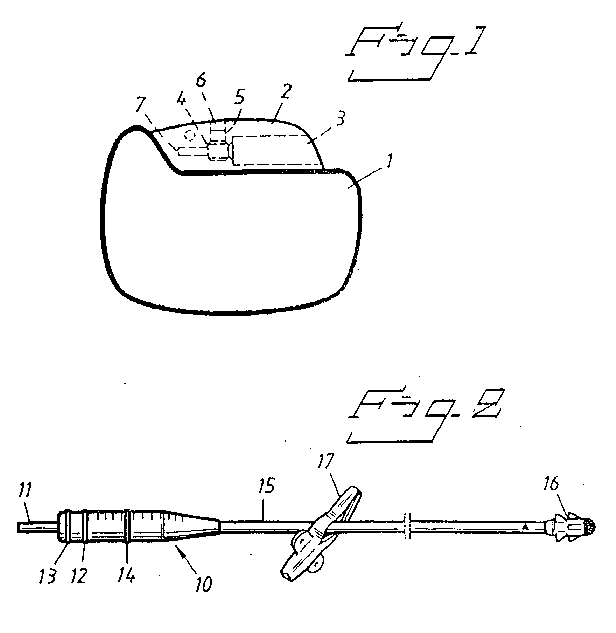

Fig 1 illustrates a conventional pacer housing 1 having a molded, transparent connective part 2. The connective part 2 includes a female socket 3. The inner end of the socket 3 is provided with a longitudinal bore 7 having a relatively small diameter. The bore 7 is provided with a contact surface 4 adjacent to which threads 5 for a set or lock screw are located in a bore 6 oriented orthogonally relative to the female socket. The housing is hermetically sealed also in relation to the molded part 2 and the contact between the interior electronics and the contact surface 4 is achieved by means of a feed-through. The feed-through comprises a ceramic plug, typically made of alumina, into which one or more leads have been soldered. This lead is bonded (e.g. ultrasonically welded) to the electronics and to the contact surface 4. The ceramic plug is soldered or brazed by means of gold into a sleeve made of titanium. This operation may be made at any time before the assembly of the pacer housing. The sleeve is welded into an opening in the housing in a sealing manner during the assembly of the pacer housing that normally consists of two halves. Before the connective part is molded onto the housing, these halves are welded together and sealed.

Fig 2 illustrates a lead 15 comprising a proximal connecting plug 10 and a distal, transvenous, intracardial electrode 16 as well as an attachment means 17 for suturing the proximal end of the lead in the body of the patient. The connecting plug 10 is designed to be received in the socket 3 and the end thereof is provided with a longitudinally projecting contact pin 11 as well as a cylindrical body provided with sealing rings 12, 13, 14 intended to engage and seal against the corresponding inner cylindrical surface of the female socket 3. The shape of the pin 11 corresponds to the shape of the bore 7. When the plug 10 is inserted into the socket 3 the pin 11 engages the contact surface 4 and the set-screw in the bore 6 can be tightened against the pin 11 in order to securely lock the plug 10 in the socket 3. The complexities involved in holding the bores, contact surfaces and threads in position and keeping them open and free from the molding material during the molding process are evident.

For the sake of simplicity, the above prior art device has been illustrated as being unipolar. A bipolar embodiment naturally will be more complex to manufacture. The preferred embodiments of the invention described below will relate to bipolar embodiments.

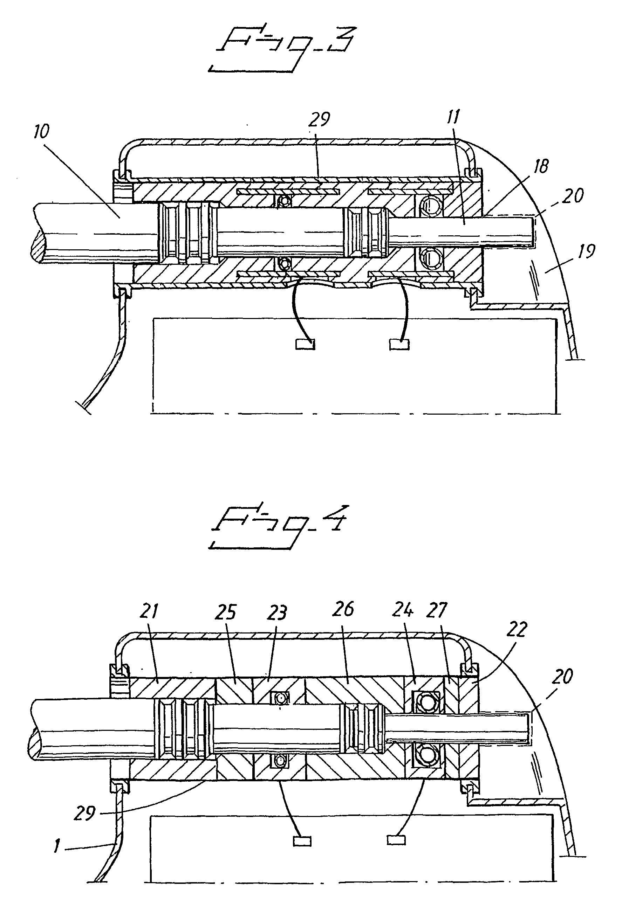

Fig 3 shows an embodiment of the invention which consequently allows for a visual confirmation that the male connector plug 10 is adequately inserted into the pulse generator. The design of the tubular connector 29 is similar as disclosed in WO 00/12174. The tubular connector 29 is in this embodiment shorter to allow the contact pin to extend beyond the second opening 18 of the tubular connector 29. A transparent component 19 is mounted on the pulse generator. The transparent component 19 is fixed to the pacer housing through e.g. glueing or other method. The transparent component 19 comprises a cavity 20 which the contact pin 11 will enter when the connector plug 10 is adequately inserted. If the connector plug 10 is adequately inserted then the contact pin 11 is visible inside the transparent component 19.

The tubular connector can of course be designed in different ways, such as for instance shown in fig 4. In this embodiment the tubular connector 29 is composed from alternating ceramic and metallic sections. The sections are brazed or soldered together using known technology. The ends 21, 22 of the tubular connector are metallic and are welded to the pacemaker encapsulation 1. The spring contacts used for connection to the connective surfaces of the contact pin are similar to the spring contacts used in US-A-9,934,366. The spring contacts are located the in metallic sections 23, 24 of the tubular connector. Ceramic sections 25, 26, 27 provide the necessary insulation to separate the spring contacts from each other and from the encapsulation.

The pacemaker also can be designed in different ways.

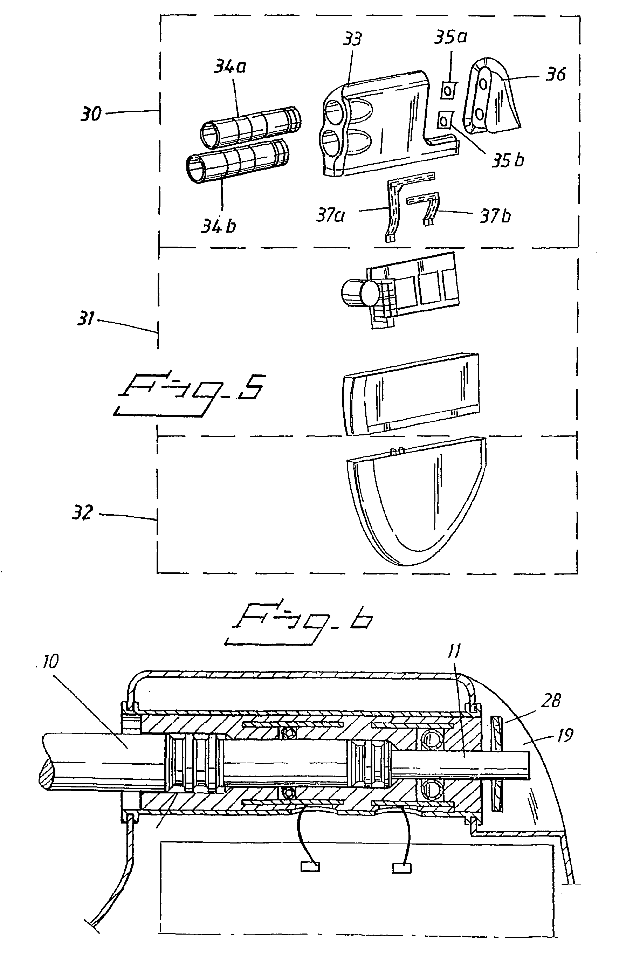

Fig 5 shows an embodiment in which the pacemaker is constructed using functional modules which contribute to the funtion as well as to the outer enclosure of the pacemaker. The pacemaker comprises at least one connector module 30, an electronics module 31 and a battery module 32. The individual modules are welded together to obtain a hermetic device with one single hermetic compartment. The connector module comprises a portion of the outer enclosure 33, the tubular connectors 34a, 34b, the locking mechanisms 35a, 35b, the transparent device 36 and flex circuits 37a, 37b for connection to the electronics module. The locking mechanisms 35a, 35b are located at the intersection between the tubular connectors 34a, 34b and the transparent device 36.

Fig 6 shows an embodiment in which the transparent component 19 comprises an electrode lead locking mechanism 28. The locking mechanism 28 is automatically activated when the contact pin 11 is advanced through said locking mechanism 28. The retention force is significantly higher than the insertion force due to bending of a portion of a washer in the locking mechanism 28.

The connector means can be achieved in a simple way compared with the prior art molded connector means while still providing a visual confirmation that the lead connector pin is adequately inserted.

The invention is applicable to any type of tubular connector that has openings in both ends. The invention is further applicable to any type of pacemaker housing that comprises a tubular connector that is welded to the pacemaker housing in both ends.

One important feature of the invention is that visual information that the connector plug is adequately inserted is available in a device without a molded connector top.

## Claims

1. An implantable heart stimulator intended to be implanted in a human body comprising an enclosure (1) and at least one tubular connector (29) having a first opening adapted to receive a heart
lead connector plug (10) comprising a contact pin (11) for electrical connection between said heart lead electrode and said heart stimulator, the ends (21,22) of said tubular connector being metallic and being welded to the enclosure (1), **characterized in that** said tubular connector (29) has a second opening allowing said contact pin (11) to extend beyond said second opening (18) of said tubular connector (29); and **in that** said
implantable heart stimulator comprises a transparent device (19) that allows visual confirmation that said connector plug (10) is adequately inserted; and that said transparent device is adapted to at least partly surround the extending part of said contact pin (11) so as to allow said visual confirmation.

2. The implantable heart stimulator according to claim 1 **characterized in that** said transparent device (19) comprises a contact pin (11) locking mechanism (28).

3. The implantable heart stimulator according to claim 1 **characterized in that** said transparent device (19) is premolded.

4. The heart stimulator according to claim 1
**characterized in that** said transparent device (19) is manufactured from a polymer material.

## Patentansprüche

1. Implantierbarer Herzstimulator, der dazu bestimmt ist, in einen menschlichen Körper implantiert zu werden, enthaltend ein Gehäuse (1) und wenigstens einen rohrförmigen Verbinder (29) mit einer ersten Öffnung, die ausgelegt ist, einen Stecker (10) einer Herzleitung aufzunehmen, der einen Kontaktstift (11) für eine elektrische Verbindung zwischen einer Elektrode der genannten Herzleitung und dem genannten Herzstimulator enthält, wobei die Enden (21, 22) des genannten rohrförmigen Verbinders metallisch und an das Gehäuse (1) angeschweißt sind, **dadurch gekennzeichnet, dass** der genannte rohrförmige Verbinder (29) eine zweite Öffnung aufweist, die es dem genannten Kontaktstift (11) ermöglicht, sich über die genannte zweite Öffnung (18) des rohrförmigen Verbinders (29) hinaus zu erstrecken; und dass der genannte implantierbare Herzstimulator eine transparente Vorrichtung (19) enthält, die eine visuelle Bestätigung dafür ermöglicht, dass der genannte Verbindungsstecker (10) adäquat eingesetzt ist; und dass die genannte transparente Vorrichtung ausgelegt ist, den vorstehenden Teil des genannten Kontaktstiftes (11) wenigstens teilweise zu umgeben, um so die genannte visuelle Bestätigung zu ermöglichen.

2. Implantierbarer Herzstimulator nach Anspruch 1, **dadurch gekennzeichnet, dass** die genannte transparente Vorrichtung (19) einen Kontaktstift (11) - Arretiermechanismus (28) enthält.

3. Implantierbarer Herzstimulator nach Anspruch 1, **dadurch gekennzeichnet, dass** die genannte transparente Vorrichtung (19) vorgeformt ist.

4. Herzstimulator nach Anspruch 1, **dadurch gekennzeichnet, dass** die genannte transparente Vorrichtung (19) aus einem Polymermaterial hergestellt ist.

## Revendications

1. Stimulateur cardiaque implantable destiné à être implanté dans un corps humain et comprenant une enceinte (1) et au moins un connecteur (29) tubulaire ayant une première ouverture conçue pour recevoir une prise (10) de connecteur de dérivation pour le coeur comprenant une broche (11) de contact de connexion électrique entre l'électrode de dérivation pour le coeur et le stimulateur cardiaque, les extrémités (21, 22) du connecteur tubulaire étant métalliques et étant soudées à l'enceinte (1), **caractérisé en ce que** le connecteur (29) tubulaire a une deuxième ouverture permettant à la broche (11) de contact de s'étendre au-delà de la deuxième ouverture (18) du connecteur (29) tubulaire ; et **en ce que** le stimulateur cardiaque implantable comprend un dispositif (19) transparent qui permet de confirmer visuellement que la prise (10) du connecteur est bien insérée ; et **en ce que** le dispositif transparent est conçu pour entourer au moins en partie la partie qui s'étend de la broche (11) de contact de manière à permettre la confirmation visuelle.

2. Stimulateur cardiaque implantable suivant la revendication 1, **caractérisé en ce que** le dispositif (19) transparent comprend un mécanisme (28) de verrouillage de la broche (11) de contact.

3. Stimulateur cardiaque implantable suivant la revendication 1, **caractérisé en ce que** le dispositif (19) transparent est prémoulé.

4. Stimulateur cardiaque suivant la revendication 1, **caractérisé en ce que** le dispositif (19) transparent est fabriqué en un matériau polymère.
